(19) Europäisches Patentamt / European Patent Office / Office européen des brevets

(11) **EP 3 004 399 B1**

(12) **EUROPÄISCHE PATENTSCHRIFT**

(45) Veröffentlichungstag und Bekanntmachung des Hinweises auf die Patenterteilung:
**15.11.2017 Patentblatt 2017/46**

(51) Int Cl.:
**C12N 1/20** *(2006.01)*     **A61K 35/747** *(2015.01)*
**C12R 1/225** *(2006.01)*

(21) Anmeldenummer: **14732517.9**

(86) Internationale Anmeldenummer:
**PCT/EP2014/061457**

(22) Anmeldetag: **03.06.2014**

(87) Internationale Veröffentlichungsnummer:
**WO 2014/195297 (11.12.2014 Gazette 2014/50)**

(54) **PROBIOTISCHE STÄMME; ZUSAMMENSETZUNGEN ENTHALTEND PROBIOTISCHE STÄMME SOWIE PHARMAZEUTISCHE MITTEL**

PROBIOTIC STRAINS, COMPOSITIONS COMPRISING THE PROBIOTIC STRAINS AND PHARMACEUTICALS

SOUCHES PROBIOTIQUES, COMPOSITIONS CONTENANTS LES SOUCHES PROBIOTIQUES ET PRODUITS PHARMACEUTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.06.2013 DE 102013105666**

(43) Veröffentlichungstag der Anmeldung:
**13.04.2016 Patentblatt 2016/15**

(73) Patentinhaber: **Hochschule Hannover**
**30539 Hannover (DE)**

(72) Erfinder:
• **PADUCH, Jan-Hendrik**
**30880 Laatzen (DE)**
• **KRÖMKER, Volker**
**32278 Kirchlengern (DE)**

(74) Vertreter: **Kröncke, Rolf**
**Gramm, Lins & Partner**
**Patent- und Rechtsanwälte PartGmbB**
**Freundallee 13 a**
**30173 Hannover (DE)**

(56) Entgegenhaltungen:
**WO-A1-2005/034970     WO-A1-2008/145756**

• **BOUCHARD DAMIEN S ET AL: "Inhibition of Staphylococcus aureus Invasion into Bovine Mammary Epithelial Cells by Contact with Live Lactobacillus casei", APPLIED AND ENVIRONMENTAL MICROBIOLOGY, Bd. 79, Nr. 3, Februar 2013 (2013-02), Seiten 877-885, XP002729767,**
• **KLOSTERMANN KATJA ET AL: "Efficacy of a teat dip containing the bacteriocin lacticin 3147 to eliminate Gram-positive pathogens associated with bovine mastitis", JOURNAL OF DAIRY RESEARCH, CAMBRIDGE UNIVERSITY PRESS, GB, Bd. 77, Nr. 2, 1. Mai 2010 (2010-05-01), Seiten 231-238, XP008172053, ISSN: 1469-7629, DOI: 10.1017/S0022029909990239 [gefunden am 2009-09-29]**

**Beschreibung**

Die Erfindung betrifft probiotische Stämme, Zusammensetzungen enthaltend mindestens zwei probiotische Stämme sowie pharmazeutische Mittel enthaltend mindestens einen erfindungsgemäßen probiotischen Stamm oder mindestens eine erfindungsgemäße Zusammensetzung.

Stand der Technik

Infektionen durch Mikroorganismen sind die wichtigste Ursache für die Entstehung von Mastitiden, bei der eine Vielzahl von Einflüssen wie Melk-, Stall- und Tierhygiene, Fütterung und gesundheitliche Kondition des Tieres zusammenwirken (Deutsche Veterinärmedizinische Gesellschaft (DVG), 2002: Leitlinien zur Bekämpfung der Mastitis des Rindes als Bestandsproblem. Gießen: DVG; Krömker V (Hrsg.): Kurzes Lehrbuch der Milchkunde und der Milchhygiene. MVS Medizinverlage, Parey, Stuttgart 2007). Die meisten mikrobiellen Erreger dringen auf dem galaktogenen Weg in die Milchdrüse ein (Deutsche Veterinärmedizinische Gesellschaft (DVG), 2002: Leitlinien zur Bekämpfung der Mastitis des Rindes als Bestandsproblem. Gießen: DVG). Aufgrund veränderter Haltungsbedingungen und durch die Anwendung neuer Maßnahmen im Rahmen des Tiergesundheitsmanagements konnte die Bedeutung der sogenannten kuhassoziierten Mastitiserreger in den letzten Jahren erheblich verringert werden (z. B. *Streptococcus (Sc.). agalactiae, Staphylococcus* (*S.*) *aureus, Sc. Dysgalactiae.* Diese genannten Mikroorganismen sind gut an die Milchdrüse und die Kuh angepasst, so dass sie vornehmlich von Kuh zu Kuh übertragen werden, was aber zu einer Zunahme von Infektionen der Milchdrüsen mit umweltassoziierten Mikroorganismen geführt hat (z. B mit *Escherichia* (*E.*) *coli, Klebsiella* spp., *Sc. uberis,* Enterokokken, Mikroorganismen, die im Haltungsumfeld der Kühe zu finden sind, wie im Einstreu, Kot). Infektionen der Milchdrüsen mit umweltassoziierten Mikroorganismen finden etwa 5,5-mal häufiger in der Trockenperiode der Tiere als in der Laktation statt (Smith et al., J.Dairy. Sci. 68: 402-417,1985). In der Trockenperiode sind vor allem die ersten Wochen nach dem Trockenstellen und die letzten Wochen vor der Abkalbung besondere Risikozeiträume. Während das Risiko einer Neuinfektion der Milchdrüse in den ersten Wochen durch die Gabe antibiotischer Langzeitpräparate in die Milchdrüsenviertel (sogenannte "Trockensteller") zumindest in konventionell wirtschaftenden Milchviehbetrieben minimiert werden kann, sind Milchkühe gegenüber Infektionen mit umweltassoziierten Mikroorganismen im abkalbenahen Zeitraum nahezu ungeschützt. Hinzu kommt, dass die Immunabwehr von Milchkühen im geburtsnahen Zeitraum sehr schwach ist (Mallard et al., J. Dairy Sci. 81 : 585-595, 1998). Neuinfektionen der Milchdrüse, die in dieser Phase stattgefunden haben, sind für etwa 60% der in der Frühlaktation stattfindenden klinischen Mastitisfälle und für 90% der schweren Mastitiden verantwortlich (Green et al., J. Dairy Sci. 85: 2589-2599, 2002; Krömker V (Hrsg.): Kurzes Lehrbuch der Milchkunde und der Milchhygiene. MVS Medizinverlage, Parey, Stuttgart 2007). Gerade durch Mastitiden, die durch Gram-negative umweltassoziierte Mikroorganismen verursacht werden, erhöht sich das Merzungsrisiko deutlich (Hertl et al., J. Dairy Sci. 94: 4863-4877, 2011).

Daneben besteht aufgrund zunehmender Antibiotikaresistenzen der Erreger die Notwendigkeit, den Antibiotikaeinsatz in der Tierhaltung zu reduzieren (Diez-Gonzalez, Curr. Issues Intestinal Microbiol. 8: 15-24, 2007). Krömker und Hamann (Der praktische Tierarzt, collegium veterinarum XXIX: 48-51, 1999) betonen vor dem Hintergrund unzureichender Wirkeffekte der antibiotischen Therapie sowie der Problematik antibiotischer Rückstände in Lebensmitteln die besondere zukünftige Bedeutung alternativer Wirkstoffe.

Vielversprechende Ansätze zur Anwendung von Schutzkulturen in der Tiergesundheit zeigen u.a. Espeche et al. (Anaerobe,18(1): 103-109, 2012). Ziel dieser Arbeit war die Bereitstellung eines probiotischen Produkts zur Prävention boviner Mastitis. Die Autoren konnten eine neue Gruppe potentiell probiotischer Stämme bereitstellen, die allein aufgrund bakterieller Eigenschaften (u.a. hohe Produktion von Hydrogenperoxid) und Sicherheit (keine Virulenz) ausgewählt wurden. *In vivo* Studien wurden nicht durchgeführt.

Die WO 2005/034970 A1 lehrt die Verwendung lebender Kulturen probiotischer Milchsäurebakterien bzw. von Überständen solcher Kulturen zur Behandlung von bakteriellen Infektionen. Insbesondere wird die Verwendung lebender Kulturen von *Lactococcus (L.) lactis* bzw. von Überständen solcher Kulturen zur Behandlung von Mastitiden offenbart. Konkret konnte *in vivo* eine Eliminierung von *S. epidermidis, S. aureus, Sc. uberis* und nicht haemolysierenden *E. coli* nach Infusion mit *L. lactis* DPC 3147 gezeigt werden, jedoch siedelten sich die Laktokokken nicht im Euter an.

Die WO 2008/145756 A1 offenbart durch ein Selektionsverfahren erhältliche probiotische Stämme bzw. Mischungen dieser Stämme, wobei das Selektionsverfahren den Schritt der Auswahl solcher Stämme umfasst, die die Überlebensrate und/oder Adhäsionsrate von S. *aureus* an epitheliale Zellen reduzieren können zur Behandlung von Infektionen, u.a. von Mastitiden. Tragende Ratten wurden jeden zweiten Tag in einem Zeitraum von 14 Tagen vor der Geburt oral mit den offenbarten Probiotika inokuliert. Bis zu 10 Tage nach der Geburt konnten diese Probiotika in den Faeces der Neugeborenen bzw. in der Muttermilch nachgewiesen werden. Eine Besiedlung der Milchdrüsenschleimhaut wurde nicht offenbart.

Im Stand der Technik sind keine probiotischen Stämme bekannt, die *in vivo* zu einer Abtötung pathogener Mikroorganismen führen und sich gleichzeitig als schützende Bakterien an Epithelien, insbesondere an solche der Milchdrüse ansiedeln.

Aufgabe der vorliegenden Erfindung ist es, probiotische Stämme bereitzustellen, die *in vivo* zu einer Hemmung pathogener Mikroorganismen führen und die sich gleichzeitig als schützende Bakterien an Epithelien, insbesondere an solche der Milchdrüse ansiedeln.

Kurze Beschreibung der Erfindung

In einem ersten Aspekt betrifft die Erfindung einen probiotischen Stamm, dadurch gekennzeichnet, dass der Stamm ausgewählt ist aus der Gruppe *Lactobacillus* sp. DSM 26911 hinterlegt bei der DSMZ unter der Eingangsnummer 26911, *Lactobacillus* sp. DSM 26912 hinterlegt bei der DSMZ unter der Eingangsnummer 26912 und *Lactobacillus* sp. DSM 26913 hinterlegt bei der DSMZ unter der Eingangsnummer 26913.

Die Erfinder haben erkannt, dass ein solcher erfindungsgemäßer Stamm in der Lage ist, *in vivo* pathogene Mikroorganismen abzutöten und sich gleichzeitig als schützend an Epithelien, insbesondere an solche der Milchdrüse ansiedelt, diese also kolonisiert.

In einem weiteren Aspekt betrifft die Erfindung eine Zusammensetzung enthaltend mindestens zwei probiotische Stämme, dadurch gekennzeichnet, dass mindestens ein Stamm davon *Lactobacillus* sp. DSM 26911 hinterlegt bei der DSMZ unter der Eingangsnummer 26911, *Lactobacillus* sp. DSM 26912 hinterlegt bei der DSMZ unter der Eingangsnummer 26912 oder *Lactobacillus* sp. DSM 26913 hinterlegt bei der DSMZ unter der Eingangsnummer 26913 ist.

In einem anderen Aspekt betrifft die Erfindung ein pharmazeutisches oder kosmetisches Mittel enthaltend mindestens einen erfindungsgemäßen probiotischen Stamm oder mindestens eine erfindungsgemäße Zusammensetzung.

Kurze Beschreibung der Figuren

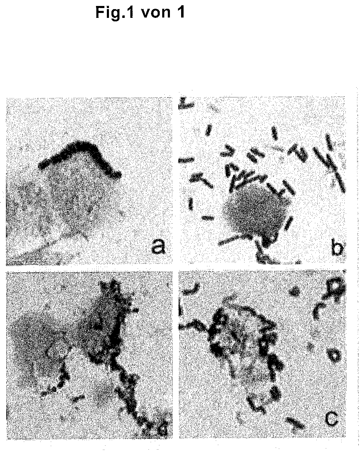

Fig. 1: Adhäsion von vier untersuchten Milchsäurebakterien (MSB) - Stämmen an Zellen des Zitzenkanalepithels (a: DSM 26911, b: DSM 26912, c: *Lb. rhamnosus* ATCC 7469, d: *Lc. lactis* ATCC 11454)

Ausführliche Beschreibung der Erfindung

Es wird ein probiotischer Stamm ausgewählt aus der Gruppe *Lactobacillus* sp. DSM 26911 hinterlegt bei der DSMZ unter der Eingangsnummer 26911, *Lactobacillus* sp. DSM 26912 hinterlegt bei der DSMZ unter der Eingangsnummer 26912 und *Lactobacillus* sp. DSM 26913 hinterlegt bei der DSMZ unter der Eingangsnummer 26913 bereitgestellt.

Überraschenderweise zeigte sich, dass ein solcher erfindungsgemäßer Stamm in der Lage ist, *in vivo* pathogene Mikroorganismen abzutöten und sich gleichzeitig als schützend an Epithelien, insbesondere an solche der Milchdrüse ansiedelt (kolonisiert).

Unter "probiotisch" im Sinne dieser Erfindung sind lebende mikrobielle, apathogene Kulturen zu verstehen, die pathogene Keime hemmen und das Wohlbefinden und/oder die Gesundheit eines Individuums (eines Menschen oder eines Tieres) positiv beeinflussen.

Als "Stamm" im Sinne dieser Erfindung wird eine bakterielle Reinkultur verstanden.

Weiterhin umfasst die Erfindung eine Zusammensetzung enthaltend mindestens zwei probiotische Stämme, dadurch gekennzeichnet, dass mindestens ein Stamm davon *Lactobacillus* sp. DSM 26911 hinterlegt bei der DSMZ unter der Hinterlegungsnummer 26911, *Lactobacillus* sp. DSM 26912 hinterlegt bei der DSMZ unter der Hinterlegungsnummer 26912 oder *Lactobacillus* sp. DSM 26913 hinterlegt bei der DSMZ unter der Hinterlegungsnummer 26913 ist. D. h. mindestens einer der mindestens zwei in der Zusammensetzung vorliegenden probiotischen Stämme ist ausgewählt aus den drei erteilungsgemäßen probiotischen Stämmen.

Unter "Zusammensetzung" ist eine Kombination umfassend zwei oder mehr probiotische Stämme zu verstehen. Die Zusammensetzung kann in flüssiger, halbfester oder fester Form vorliegen. Weitere Bestandteile können beispielsweise Nährmedien, Nukleotide, Vitamine, Fettsäuren, Lipide, Aminosäuren, Proteine, Mono- sowie Polysaccharide, Wasser, Öle, Glycerol etc. umfassen.

Vorzugsweise sind der oder die weiteren Stämme mindestens einer aus *Lactococcus lactis* ATCC 11454 und *Lactobacillus rhamnosus* ATCC 7469. Der Effekt solcher Zusammensetzungen ist, dass insbesondere Mastitiserreger, die bei Rindermastitiden als Kuh- und/oder Umwelt-assoziiert zusammengefasst werden, gehemmt werden können.

In einer bevorzugten Ausführungsform enthält die Zusammensetzung die probiotischen Stämme *Lactococcus lactis* ATCC 11454 und *Lactobacillus* sp. DSM 26911. Es wurde festgestellt, dass diese Zusammensetzung besonders gut für eine Hemmung von *S. epidermidis, S. xylosus* und *Sc. uberis* geeignet ist.

In einer weiteren bevorzugten Ausführungsform enthält die Zusammensetzung die probiotischen Stämme *Lactococcus lactis* ATCC 11454 und *Lactobacillus* sp. DSM 26912. Diese Zusammensetzung zeigt insbesondere eine Hemmung von S. aureus, S. epidermidis, *Sc. uberis* und *Sc. agalactiae.*

In einer Weiterbildung der Erfindung enthält die Zusammensetzung die probiotischen Stämme *Lactobacillus rhamnosus* ATCC 7469 und *Lactobacillus* sp. DSM 26911. Es wurde festgestellt, dass diese Zusammensetzung besonders gut für eine Hemmung von S. *epidermidis* und *Sc. uberis* geeignet ist.

In einer besonderen Ausführungsform enthält die Zusammensetzung die probiotischen Stämme *Lactobacillus rhamnosus* ATCC 7469 und *Lactobacillus* sp. DSM 26912. Diese Zusammensetzung führt insbesondere zu einer Hemmung von S. *epidermidis, Sc. uberis* und *E. coli.*

In einer anderen Weiterbildung der Erfindung enthält die Zusammensetzung die probiotischen Stämme *Lactobacillus* sp. DSM 26911 und *Lactobacillus* sp. DSM 26912. Effekt ist insbesondere eine Hemmung von S. *epidermidis* und *Sc. uberis.*

In einer bevorzugten Ausführungsform enthält die Zusammensetzung die probiotischen Stämme *Lactobacillus* sp. DSM 26911, *Lactobacillus* sp. DSM 26912, *Lactobacillus rhamnosus* ATCC 7469 und *Lactococcus lactis* ATCC 11454. Überraschenderweise zeigte die Zusammensetzung eine besonders gute Hemmung von *Sc. uberis, S. aureus, S. epidermidis, S.xylosus, Sc. agalactiae* und *E.coli.*

Weiterhin betrifft die Erfindung ein pharmazeutisches Mittel enthaltend mindestens einen erfindungsgemäßen probiotischen Stamm oder mindestens eine erfindungsgemäße Zusammensetzung.

Insbesondere ist das pharmazeutische Mittel formuliert für die Prophylaxe und/oder Therapie einer chronischen oder akuten Infektion oder unerwünschten mikrobiellen Kolonisation, wobei die Infektion oder Kolonisation durch Mikroorganismen, insbesondere durch Bakterien, aber auch durch Parasiten, Pilze, Hefen oder Viren verursacht bzw. erfolgt ist. Eine solche Infektion oder Kolonisation kann jede Körperoberfläche, einschließlich der von Schleimhäuten ausgekleideten Hohlorgane, wie beispielsweise den Darm, oder auch beispielsweise die Milchdrüse, betreffen.

Vorzugsweise ist das pharmazeutische Mittel formuliert für die Verwendung in der Prophylaxe und/oder Therapie von Mastitis.

In einer anderen Weiterbildung der Erfindung umfasst das pharmazeutische Mittel einen Träger, wie beispielsweise ein textiles Gewebe. Diese Ausführungsform erleichtert insbesondere die Versorgung der menschlichen Brustdrüse.

In einer weiteren Ausführungsform umfasst das pharmazeutische Mittel eine Matrix, die eine intramammäre Anwendung des Mittels erlaubt, beispielsweise eine Salbe oder einen Zitzenstift. Diese Ausführungsform ist besonders für die Versorgung der Milchdrüse des weiblichen Rindes geeignet.

Das pharmazeutische Mittel kann als applizierbare Lösung oder Creme formuliert sein. Vorzugsweise ist das pharmazeutische Mittel als Zitzentauchmittel oder als Sprühmittel formuliert. Eine solche Formulierung erlaubt eine besonders einfache und schonende Versorgung der Milchdrüse. Das Zitzentauchmittel umfasst vorzugsweise MRS-Bouillon (z.B. nach de Man et al., J. Appl. Bact. 23: 130-135, 1960). Weiter vorzugsweise ist ein Chlorophyllin-Kupfer-Komplex oder ein anderer geeigneter Farbstoff zur effektiven Visualisierung der Anwendung enthalten, insbesondere kann weiterhin Magermilchpulver, vorzugsweise in Anteilen von 0,5 %, 1,0 %, 1,5 % und 2,0 %, bezogen auf das Gesamtvolumen enthalten sein.

Weiter vorzugsweise ist das pharmazeutische Mittel als Zitzentauchmittel für die Trockenperiode des Rindes formuliert. Eine solche Formulierung bietet den Vorteil, dass insbesondere die in der Frühlaktation auftretenden Mastitiden zuverlässig verhindert werden können.

In einer besonderen Ausführungsform ist das pharmazeutische Mittel für das präpartale Trächtigkeitsstadium formuliert.

Erfindungsgemäß umfasst ein Verfahren zur Prophylaxe und/oder Therapie von unerwünschten mikrobiellen Infektionen oder Kolonisationen die Verabreichung einer pharmazeutisch aktiven Dosis mindestens eines erfindungsgemäßen probiotischen Stammes oder mindestens einer erfindungsgemäßen Zusammensetzung. Insbesondere ist ein solches Verfahren zur Prophylaxe und/oder Therapie von akuter oder chronischer Mastitis, insbesondere bei Rindern, vorzugsweise in der Trockenperiode, weiter vorzugsweise in der präpartalen Trockenperiode, geeignet.

Das erfindungsgemäße pharmazeutische Mittel kann lokal, enteral oder parenteral appliziert werden. Eine enterale Verabreichung ist insbesondere als orale und rektale Applikation möglich, eine lokale Verabreichung kann unter anderem dermal, vaginal und intramammär erfolgen. Die Wirkung ist insbesondere topisch. Das pharmazeutische Mittel kann vorteilhafterweise instilliert werden. Bevorzugt erfolgt die Applikation intramammär oder dermal im Bereich der Milchdrüse. Das pharmazeutische Mittel kann optional pharmazeutisch akzeptable Träger, Verdünnungsmittel und/oder Zusatzstoffe enthalten.

Vorteilhafterweise liegen die im pharmazeutischen Mittel enthaltenen Mikroorganismen in gefrorener, lyophylisierter oder getrockneter Form vor. Das pharmazeutische Mittel wird dabei erst unmittelbar vor der Anmeldung in die gewünschte Applikationsform, z.B. als Zitzentauchmittel oder Sprühmittel, überführt. Zum einen vermindert die Zubereitung des pharmazeutischen Mittels unmittelbar vor der Anwendung *in praxi* die Gefahr möglicher Kontaminationen mit anderen Mikroorganismen, zum anderen können durch das Gefrieren, das Lyophylisieren oder das Trocknen der Mikroorganismen hohe Keimdichten in Verbindung mit einer hohen Lagerstabilität realisiert werden. Methoden, insbesondere zur Gefriertrocknung von lebensmitteltechnologisch relevanten Mikroorganismen (Starter-, Reifungskulturen) sind in der Praxis bereits etabliert. In dieser vorteilhaften Ausführungsform kann das Mittel neben den gefrorenen, lyophylisierten oder getrockneten Mikroorgansimen einen geeigneten Träger, wie entsprechende Grundlagen für die gewünschte Applikationsform, z.B. ein geeignetes Medium, beispielsweise MRS-Bouillon (nach de Man et al., J. Appl. Bact. 23: 130-135, 1960), umfassen, in dem die Mikroorganismen suspendiert werden können.

Die Erfindung wird weiter unter Bezugnahme auf die Figuren und die Ausführungsformen ausgeführt. Dies soll der Veranschaulichung der Erfindung, nicht jedoch ihrer Beschränkung dienen.

**Versuche**

*In vitro* Hemmung

In *in vitro* Versuchen wurde die Hemmwirkung ausgewählter kommerziell erhältlicher probiotischer Stämme (Schutzkulturen) auf ausgewählte Mastitiserreger untersucht. Zusätzlich wurden im Rahmen der Versuche aus vier Gülle- und 181 Rohmilchproben sowie aus 80 Tupferproben der Zitzenhaut und des Zitzenkanals von Milchkühen Milchsäurebakterien (MSB)-Stämme isoliert und die Isolate ebenfalls hinsichtlich hemmender Eigenschaften gegenüber Mastitiserregern geprüft. Als Milchsäurebakterien wurden dabei Grampositive, kokken- oder stäbchenförmige, nichtsporenbildende, Katalase-, Oxidase-, $H_2S$- und Indol-negative und unbewegliche Bakterien eingestuft, welche über einen fermentativen Stoffwechsel verfügen (Garvie EI (1986 a): Genus Leuconostoc. In: Sneath, P. H. A. (editor). Bergey's Manual of Systematic Bacteriology. Volume 2. Baltimore, Hong Kong, London, München, Philadelphia, San Francisco, Sydney, Tokyo: 1071-1075; Garvie EI (1986 b): Genus Pediococcus. In: Sneath, P. H. A. (editor). Bergey's Manual of Systematic Bacteriology. Volume 2. Baltimore, Hong Kong, London, München, Philadelphia, San Francisco, Sydney, Tokyo: 1075-1079; Hardie, JM (1986): Genus Streptococcus. In: Sneath, P. H. A. (editor). Bergey's Manual of Systematic Bacteriology. Volume 2. Baltimore, Hong Kong, London, München, Philadelphia, San Francisco, Sydney, Tokyo: 1043-1071 ; Kandler O, Weiss N (1986): Genus Lactobacillus. In: Sneath, P. H. A. (editor). Bergey's Manual of Systematic Bacteriology. Volume 2. Baltimore, Hong Kong, London, München, Philadelphia, San Francisco, Sydney, Tokyo: 1209-1234). MSB-Isolate, die hemmende Eigenschaften auf das Wachstum der Infektionserreger aufwiesen, wurden anschließend mit dem API-System anhand biochemischer Eigenschaften auf Speziesebene differenziert.

Darüber hinaus wurden Kombinationen von Schutzkulturen auf ihren Hemmeffekt gegenüber pathogenen Mikroorganismen untersucht.

Im Rahmen der Untersuchungen wurde der Well-Diffusionstest in Anlehnung an Ryan et al. (Appl. Environ. Microbiol. 62: 612-619, 1996 und Appl. Environ. Microbiol. 64: 2287-2290, 1998) durchgeführt.

Als euterpathogene Indikatororganismen wurden die Referenzstämme S. *aureus* ATCC 12600, *Sc. agalactiae* ATCC 27956, *Sc. uberis* ATCC 700407 und *E. coli* DSM 4230 sowie die beiden aus Rohmilchproben isolierten Stämme S. *epidermidis* 575/08 und *S. xylosus* 35/07 verwendet. Die Anzucht der Indikatororganismen erfolgte in Hirn-Herz-Bouillon (Merck) bei 37°C für 24 h. Für die Anzucht der MSB-Stämme wurde MRS-Bouillon (pH 6,2; AppliChem) verwendet. Die Inkubation der MSB-Stämme erfolgte bei 37°C für 48-72 h. Als Nährmedium für den Well-Diffusionstest wurde für die Indikatororganismen Müller-Hinton-Agar (Merck) und für die MSB-Stämme MRS-Agar (pH 6,2; AppliChem) verwendet. 20 ml des jeweiligen Agars wurden mit einer Subkultur der Bakterien-Stämme (ca. $2,0 \times 10^6$-$4,0 \times 10^8$ KbE/ml) beimpft, und anschließend je Platte fünf Wells mit einem Durchmesser von 6 mm in den Agar gestanzt. Zur Unterstützung der Diffusion wurden die Platten vor der Inkubation zunächst bei 7°C für 16 h gelagert (Mathot et al., J. Dairy Sci. 86: 3068-3074, 2003). Hemmzonen mit einer Breite $\geq 2$ mm wurden bei der Auswertung der Platten als "Hemmung" gewertet. Dabei war die Breite der Hemmzone die Distanz zwischen der Außenkante des Wells und der äußeren Grenze der Hemmzone.

a) Für das Screening der MSB-Stämme hinsichtlich ihrer Hemmwirkung gegenüber den Indikatororganismen wurden 6 ml einer inkubierten MRS-Anreicherungsbouillon (37°C/72 h) bei 8.200 x g für 5 min zentrifugiert und der Überstand sterilfiltriert (Minisart, 0,2 $\mu$m; Satorius). Jeweils 50$\mu$l des Filtrats wurden in die Wells gegeben. Die Inkubation der Müller-Hinton-Platten erfolgte bei 37°C bzw. parallel bei 25°C über 24h.

b) Für das Screening der MSB-Stämme hinsichtlich einer gegenseitigen Hemmwirkung wurden 2 ml einer inkubierten MRS-Bouillon (37°C/72 h) bei 8.200 x g für 5 min zentrifugiert, sterilfiltriert und 50 $\mu$l des zellfreien Filtrates in vorbereitete Wells der beimpften MRS-Agar-Platten pipettiert. Die Bebrütung der Agarplatten erfolgte bei 37°C für 72 h. Dabei sollte ermittelt werden, ob diese Schutzkulturen eine Hemmwirkung gegenüber den Indikatororganismen zeigen.

c) Auf Basis der ermittelten Daten aus a) und b) wurden anschließend Kombinationen von Schutzkulturen in Hinblick auf deren Hemmeffekt gegenüber den Indikatororganismen untersucht. Dafür wurden von jeweils zwei bis vier entsprechend inkubierten MRS-Bouillons (37°C/72 h) 100 μl aus den Anreicherungen vermischt und 50 μl aus dieser Zellsuspension im Well-Diffusionstests eingesetzt. Die Bebrütung der Müller-Hinton-Platten erfolgte bei 37°C für 24 h.

Überlebensfähigkeit der probiotischen Stämme in verschiedenen Lösungen zur Ermittlung einer geeigneten Zitzentauchmittelgrundlage

Die Überlebensfähigkeit der MSB-Kulturen (als Teststämme dienten *Lb. rhamnosus* ATCC 7469, *Lc. lactis* ATCC 11454, und die Isolate 78/37 -nach der Hinterlegung bei der DSMZ als *Lactobacillus sp.* DSM 26911 bezeichnet- und 118/37 -nach der Hinterlegung bei der DSMZ als *Lactobacillus sp.* DSM 26912 bezeichnet-) wurde zunächst in mehreren verschiedenen Lösungen (sterile, ¼ starker Ringerlösung (Merck) mit unterschiedlichen Konzentrationen von Glycerol (5,0 %, 3,0 % 2,5 %, 2,0 %, 1,5 %, 1,0 % (v/v)), Lebensmittelfarbe-Blau (2,0 %, 1,0 %, 0,5 %, 0,4 %, 0,3 %, 0,2 %, 0,1 % (v/v)), Lebensmittelfarbe-Grün (2,0 %, 1,0 %, 0,5 %, 0,4 %, 0,3 %, 0,2 %, 0,1 % (v/v)) oder Chlorophyllin-Kupfer-Komplex (0,5 %, 0,1 %, 0,075 %, 0,05 % (w/v)) getestet. Die Teststämme wurden dazu vorher in MRS-Bouillon (pH 5,5; AppliChem) bei 37°C für 72 h angereichert und anschließend der Keimgehalt jeder Bouillon im Overlayverfahren bestimmt. Jeweils 1 ml der inkubierten MRS-Bouillon (37°C/72 h) wurde bei 8.200 x g zentrifugiert und der Überstand verworfen. Das Zellpellet wurde anschließend in 1 ml der jeweils zu testenden Lösung resuspendiert und der Keimgehalt aus dieser Suspension an Tag 0 (Ausgangskeimgehalt), Tag 1, Tag 2, Tag 3 und Tag 4 bestimmt. Die Lagerung der die Teststämme enthaltenden Lösungen erfolgte über den Versuchszeitraum bei Raumtemperatur. Als Nährmedium zur Keimzahlbestimmung wurde MRS-Agar (pH 6,2; AppliChem) verwendet. Die Bebrütung der Platten erfolgte bei 37°C für 48 h.

Auf Basis der Erkenntnisse aus dem geschilderten Versuch zur Überlebensfähigkeit der Teststämme wurde als eine mögliche Zitzentauchmittelrezeptur eine inkubierte MRS-Bouillon (37°C/72 h) mit einem Zusatz von 0,1 % Chlorophyllin-Kupfer-Komplex (Caelo) (w/v) (siehe Rezeptur 1001) aufgrund der besten Überlebensfähigkeit der Teststämme in der entsprechenden Lösung aus ¼ starker Ringerlösung und 0,1% (w/v) Chlorophyllin-Kupfer-Komplex (Caelo), hergestellt.

Zusätzlich sollte überprüft werden, ob ein Zusatz von Magermilchpulver die Überlebensfähigkeit der Schutzkulturen weiter verbessert. Hierfür wurde der Zitzentauchmittelrezeptur 1001 Magermilchpulver in Anteilen von jeweils 0,5 %, 1,0 %, 1,5 % und 2,0 % zugesetzt (siehe Rezepturen 1002-1005).

Die probiotischen Stämme wurden jeweils in 50 ml MRS-Bouillon (pH 5,5) bei 37°C für 72 h inkubiert, anschließend 25 ml aus dieser Anreicherung bei 900 x g für 10 min zentrifugiert und der Überstand verworfen. Die Zellen wurden in den verbleibenden 25 ml der inkubierten MRS-Bouillon resuspendiert und 7,9 ml davon für die Herstellung der verschiedenen Zitzentauchmittel-Rezepturen mit unterschiedlichen Gehalten an Milchpulver verwendet. Die 10 %ige Chlorophyllin-Kupfer-Komplex-Lösung (w/v) wurde vor der Verwendung sterilfiltriert, die 10 %ige Magermilchpulverlösung wurde aseptisch mit sterilem destilliertem Wasser (Aqua$_{dest.}$) hergestellt.

Zusammensetzung der verschiedenen Zitzentauchmittel-Rezepturen (als Rezepturen 1001-1005 bezeichnet):

| Rezeptur 1001 : | ohne Zusatz von Magermilchpulver: |
| | 7,9 ml inkubierte MRS-Bouillon |
| | 0,1 ml 10 %ige Chlorophyllin-Kupfer-Komplex-Lösung |
| | 2,0 ml Aqua$_{dest.}$ |

| Rezeptur 1002: | 2,0 % Magermilchpulverzusatz: |
| | 7,9 ml inkubierte MRS-Bouillon |
| | 0,1 ml 10 %ige Chlorophyllin-Kupfer-Komplex-Lösung |
| | 2,0 ml 10 %ige Magermilchpulver-Lösung |

| Rezeptur 1003: | 1,5 % Magermilchpulverzusatz: |
| | 7,9 ml inkubierte MRS-Bouillon |
| | 0,1 ml 10 %ige Chlorophyllin-Kupfer-Komplex-Lösung |
| | 1,5 ml 10 %ige Magermilchpulver-Lösung |
| | 0,5 ml Aqua$_{dest.}$ |

| Rezeptur 1004 | 1,0 % Magermilchpulverzusatz: |
| | 7,9 ml inkubierte MRS-Bouillon |
| | 0,1 ml 10 %ige Chlorophyllin-Kupfer-Komplex-Lösung |
| | 1,0 ml 10 %ige Magermilchpulver-Lösung |

(fortgesetzt)

1,0 ml Aqua$_{dest.}$

| Rezeptur 1005 | 0,5 % Magermilchpulverzusatz: |
| | 7,9 ml inkubierte MRS-Bouillon |
| | 0,1 ml 10 %ige Chlorophyllin-Kupfer-Komplex-Lösung |
| | 0,5 ml 10 %ige Magermilchpulver-Lösung |
| | 1,5 ml Aqua$_{dest.}$ |

Adhäsion von Milchsäurebakterien an das Zitzenkanalepithel

*In vivo*

In zwei Versuchsreihen wurde ermittelt, ob eine Etablierung der MSB-Stämme auf der Zitzenhaut laktierender und trockenstehender Kühe gelingt. Die Zitzenhaut von fünf laktierenden bzw. acht trockenstehenden klinisch gesunden Kühen wurde mit Flüssigkulturen der vier Stämme *Lactobacillus sp.* DSM 26911, *Lactobacillus sp.* DSM 26912, *Lb. rhamnosus* ATCC 7469 und *Lc. lactis* ATCC 11454 besprüht. Die Zitzen wurden zuvor mit einem Einwegtuch gereinigt und mit 70%igen Ethanol desinfiziert.

An Tag 1, Tag 2, Tag 3, Tag 4 und Tag 5 (laktierende Tiere) bzw. an Tag 7 und Tag 14 (trockenstehende Tiere) wurde mit dem modifizierten Nass-Trocken-Tupfer-Verfahren nach DIN 10113-1: 1997-07 (Paduch und Krömker, Tierärztl Prax 39 (G): 71-76, 2011) die Zitzenhaut beprobt. Das jeweilige Tupferpaar wurde in Röhrchen mit 2,5 ml steriler, ¼ starker Ringerlösung überführt. Nachdem die Proben für 20 sec homogenisiert wurden, wurde die Keimzahl mit MRS-Agar (AppliChem, pH 5,5) bestimmt.

*In vitro*

Ergänzend wurde die Adhäsion von Milchsäurebakterien an Epithelzellen in Anlehnung an Frola et al. (J. Dairy Res. 14:1-9, 2011) untersucht. Dazu wurden die Stämme in 20 ml MRS-Bouillon (pH 5,5) bei 37°C für 48-72h angezüchtet und zweimal subkultiviert. 10 ml der jeweiligen inkubierten Bouillon wurden bei 900 x g für 10 min zentrifugiert (Hettich, Typ 4210), der Überstand verworfen und zweimal mit 5 ml steriler 0,8 % NaCl-Lösung (w/v) und einmal mit 5 ml MEM-Medium (Gibco, pH 7,0) gewaschen. Das Zellpellet wurde nach der Zentrifugation in 2 ml MEM resuspendiert. Die Keimdichte wurde auf ca. $10^7$ KbE/ml eingestellt. Zellen des Zitzenkanalepithels wurden aus frischen Schlachtzitzen gewonnen und sofort in 10 ml MEM (pH 7,0) suspendiert. Die Zellen wurden dreimal mit 5 ml MEM gewaschen und das Zellpellet nach der Zentrifugation in 2 ml MEM resuspendiert. Eine Ausgangskonzentration von $10^5$ Zellen/ml wurde eingestellt. Jeweils 500 $\mu$l der MSB-Suspension und der Epithelzellensuspension wurden gemischt und unter leichtem Schütteln bei 37 °C für 1 h inkubiert. Dieinkubierte Suspension wurde bei 900 x g für 10 min zentrifugiert (Spectrafuge 16M) und das Zellpellet viermal mit 1 ml MEM gewaschen. Die Adhäsion der Bakterienzellen an die Epithelzellen wurde mikroskopisch bestimmt; die folgenden Kenngrößen wurden ermittelt:

Adhäsion [%] = Anzahl der Epithelzellen mit anhaftenden Bakterien / Anzahl der gesamten Epithelzellen x 100

Adhäsionsindex = Anzahl der anhaftenden Bakterien an Epithelzellen / Anzahl der Epithelzellen mit anhaftenden Bakterien

Prophylaktische Wirkung gegen Mastitis (Feldversuch)

Als Versuchsbetriebe zur Testung des Zitzentauchmittels (Zusammensetzung des Zitzentauchmittels siehe unter Ergebnisse, Prophylaktische Wirkung gegen Mastitis (Feldversuch)) wurden fünf Milchviehbetriebe ausgewählt (zwei Betriebe in Brandenburg, je einer in Sachsen-Anhalt, Niedersachsen und Nordrhein-Westfalen). Die Herden wiesen Größen zwischen 154 und 1539 laktierende Kühen auf und erzielen durchschnittliche Laktationsleistungen von 7300 bis 11176 kg Milch pro Kuh. Alle Betriebe zeichneten sich durch eine überdurchschnittlich hohe Färsenmastitisrate (% Färsen von allen Färsen eines Jahres, die in ihrer ersten Milchkontrolle > 100.000 Zellen/ml aufweisen) von über 40% aus. Zwischen 41 und 61 % der Färsen dieser Betriebe wiesen bei der ersten Milchleistungsprüfung nach der Abkalbung einen somatischen Zellgehalt von mehr als 100.000 Zellen pro ml Milch auf.

Die Färsen der Anfütterungsgruppen wurden in einem Zeitraum von etwa zwei Wochen vor dem errechneten Geburtstermin einmal wöchentlich mit dem gekühlt transportierten Zitzentauchmittel gedippt (d.h. die Zitzen der Färsen wurden kurz in das Zitzentauchmittel eingetaucht). Es wurden nur klinisch gesunde Färsen mit vier funktionsfähig erscheinenden Zitzen ohne Verletzungen oder Spuren älterer Verletzungen in den Versuch aufgenommen. Das Dippen erfolgte in einem Split-Udder-Design mittels eines konventionellen Dippbechers. Die linke Vorderzitze und die rechte Hinterzitze wurden mit dem Zitzentauchmittel benetzt.

Andere pharmazeutische Mittel zur Vorbeugung von Färsenmastitiden wurden auf den Versuchsbetrieben nicht eingesetzt.

In der ersten Woche nach dem Abkalben sowie in der darauffolgenden Woche wurden von jeder gedippten Färse Viertelanfangsgemelksdoppelproben aller Euterviertel aseptisch entnommen. Dabei wurden die "Leitlinien Entnahme von Milchproben unter aseptischen Bedingungen und Isolierung und Identifizierung von Mastitiserregern" der Deutschen Veterinärmedizinischen Gesellschaft berücksichtigt (DVG, 2009, Gießen: DVG).

Die Milchproben wurden am Tag der Entnahme in das mikrobiologische Labor der Hochschule Hannover gebracht und sofort mit der durch die DVG empfohlenen Untersuchungsmethodik (Deutsche Veterinärmedizinische Gesellschaft (DVG), 2009: Leitlinien Entnahme von Milchproben unter aseptischen Bedingungen und Isolierung und Identifizierung von Mastitiserregern. Gießen: DVG) untersucht. Eine Probe wurde als kontaminiert eingestuft, wenn mehr als zwei verschiedene Kolonietypen isoliert wurden. Der Gehalt somatischer Zellen wurde durchflusszytometrisch mit dem Gerät Somascope Smart (Cv < 5 %) bestimmt. Die Zuordnung der einzelnen Euterviertel zu den Mastitiskategorien (normale Sekretion, latente Infektion, unspezifische Mastitis, Mastitis) erfolgte auf Basis des Ergebnisses der zytobakteriologischen Untersuchung (Deutsche Veterinärmedizinische Gesellschaft (DVG), 2002: Leitlinien zur Bekämpfung der Mastitis des Rindes als Bestandsproblem. Gießen: DVG). Das Auftreten klinischer Mastitisfälle sowie die Ergebnisse der Milchleistungsprüfungen der behandelten Tiere in den ersten 100 Laktationstagen wurden dokumentiert.

Mit dem entwickelten Zitzentauchmittel wurden Färsen in den letzten zwei Wochen vor der Abkalbung behandelt. Paduch und Krömker (Tierärztl Prax 39 (G): 71-76, 2011) konnten bei primiparen und multiparen Tiere eine hohe Variationsbreite der Keimdichten euterpathogener Mikroorgansimen auf den Zitzenepithelien laktierender Tiere feststellen. Dies kann möglicherweise auf eine Vielzahl prädisponierender Faktoren für die mikrobielle Besiedlung der Zitzenhaut und des Zitzenkanals in Milchviehbetrieben zurückgeführt werden. Um die Bedeutung möglicher Einflussfakoren (z. B. Zitzenepithelschädigungen durch Infektionen oder Milchentzug in vorhergehenden Laktationen) zu reduzieren, wurden daher ausschließlich Färsen, deren Behandlung in einem Split-Udder-Versuch erfolgte, einbezogen.

## Ergebnisse

### *In vitro* Hemmung

Tabelle 1: Ergebnisse der *in vitro* Versuche zur Hemmung euterpathogener und fakultativ euterpathogener Mikroorganismen durch Einzelstämme und Kombinationen von Milchsäurebakterien (Well-Diffusionstest, Hemmzonen in mm, eine Hemmung wurde ab ≥2 mm als Hemmung definiert)

| Stammbezeichnung/ Kombinationen | Hemmzonen [mm] | | | | | |
|---|---|---|---|---|---|---|
| | *S. aureus* ATCC 12600 | *S. epidermidis* 575/08 | *S. xylosus* 35/07 | *Sc. uberis* ATCC 700407 | *Sc. agalactiae* ATCC 27956 | *E. coli* DSM 4230 |
| Isolat 78/37[1] | 0 | **2** | 1 | 0 | 0 | n.a.* |
| Isolat 118/37[2] | 0 | **6** | 0 | 1 | 0 | n.a.* |
| Isolat 136/37[3] | 0 | **4** | 0 | 0 | 0 | **9** |
| *Lb. rhamnosus* ATCC 7469 | 0 | **3** | 2 | 1 | 0 | n.a.* |
| *Lc. lactis* ATCC 11454 | 0 | **6** | 2 | 0 | **6** | **2** |
| Isolat 78/37[1] + Isolat 118/37[2] | 0 | **5** | 0 | **2** | 0 | 0 |

(fortgesetzt)

| Stammbezeichnung/ Kombinationen | Hemmzonen [mm] | | | | | |
|---|---|---|---|---|---|---|
| | *S. aureus* ATCC 12600 | *S. epidermidis* 575/08 | *S. xylosus* 35/07 | *Sc. uberis* ATCC 700407 | *Sc. agalactiae* ATCC 27956 | *E. coli* DSM 4230 |
| Isolat 78/37[1] + *Lb. rhamnosus* ATCC 7469 | 0 | **3** | 0 | **2** | 0 | 0 |
| Isolat 78/37[1] + *Lc. lactis* ATCC 11454 | 0 | **2** | **2** | **2** | 0 | 0 |
| Isolat 118/37[2] + *Lb. rhamnosus* ATCC 7469 | 0 | **2** | 0 | **2** | 0 | **2** |
| Isolat 118/37[2] + *Lc. lactis* ATCC 11454 | **2** | **2** | 0 | **2** | **4** | 0 |
| *Lc. lactis* ATCC 11454 + *Lb. rhamnosus* ATCC 7469 | 0 | **5** | 0 | 0 | **2** | 0 |
| Isolat 78/37[1] + Isolat 118/37[2] + *Lb. rhamnosus* ATCC 7469 + *Lc. lactis* ATCC 11454 | **2** | **5** | **2** | **2** | **3** | **2** |
| * nicht auswertbar: eine Hemmung war zu beobachten, die Hemmzonen waren jedoch nicht deutlich ausgeprägt<br>[1] nach der Hinterlegung bei DSMZ als *Lactobacillus sp.* DSM 26911 bezeichnet<br>[2] nach der Hinterlegung bei DSMZ als *Lactobacillus sp.* DSM 26912 bezeichnet<br>[3] nach der Hinterlegung bei DSMZ als *Lactobacillus sp.* DSM 26913 bezeichnet | | | | | | |

Ergebnisse von Einzelstämmen oder von Kombinationen zweier Stämme:

Während *Staphylococcus* (*S.*) *aureus* ATCC 12600 nur durch die Kombination aus den Stämmen 118/37 und *Lactococcus* (*Lc.*) *lactis* ATCC 11454 sowie S. *xylosus* 35/07 durch die Einzelstämme *Lactobacillus* (*Lb.*) *rhamnosus* ATCC 7469 und *Lc. lactis* ATCC 11454 sowie die Kombination aus 78/37 und *Lc. lactis* ATCC 11454 wirksam gehemmt wurden, hemmten alle untersuchten Einzelstämme und Zweierkombinationen S. *epidermidis* 575/08. Eine Hemmung von *Streptococcus* (*Sc.*) *uberis* ATCC 700407 konnte mit Ausnahme der Kombination aus *Lc. lactis* ATCC 11454 und *Lb. rhamnosus* ATCC 7469 durch alle getesteten Zweierkombinationen realisiert werden. *Sc. agalactiae* ATCC 27956 wurde durch den Einzelstamm *Lc. lactis* ATCC 11454 sowie durch die Kombinationen aus 118/37 und *Lc. lactis* ATCC 11454 und aus *Lc. lactis* ATCC 11454 und *Lb. rhamnosus* ATCC 7469 gehemmt. Der Einzelstamm *Lc. lactis* ATCC 11454 sowie der Einzelstamm 136/37 und die Kombination aus 118/37 und *Lb. rhamnosus* ATCC 7469 hemmten *Escherichia coli* DSM 4230.

Ergebnis der Kombination von vier Stämmen:

Durch die Kombination aller vier Stämme (*Lactobacillus* (*Lb.*) *rhamnosus* ATCC 7469, *Lc. lactis* ATCC 11454, Stamm (Isolat) 78/37 und Stamm (Isolat) 118/37) konnte eine Hemmung aller in die Untersuchungen einbezogenen euterpathogenen und fakultativ euterpathogenen Mikroorganismen erzielt werden.
Aus den Ergebnissen lassen sich folgende Schlussfolgerungen ableiten:

• Durch die Kombination verschiedener Stämme konnte das Wirkspektrum der getesteten Einzelstämme deutlich erweitert werden, so dass auch eine Hemmung des bedeutenden kontagiösen Mastitiserregers S. *aureus,* der in dieser Untersuchung durch keinen Einzelstamm gehemmt wurde, realisiert werden konnte.
• Alle in die Untersuchungen einbezogenen kuh- und umweltassoziierten euterpathogenen oder fakultativ euterpathogenen Mikroorganismen konnten durch mindestens eine der Kombinationen gehemmt werden.
• Durch die Kombination aller vier Einzelstämme können sowohl kuh- als auch umweltassoziierte Mastitiserreger gehemmt werden.

Die Auswertung der Untersuchung einer möglichen gegenseitigen Hemmung der MSB-Stämme zeigte, dass die einzelnen MSB-Kulturen untereinander keinen wachstumshemmenden Einfluss ausübten.

Auswahl einer geeigneten Zitzentauchmittelrezeptur:

Der Ausgangskeimgehalt in den jeweiligen Rezepturen aus MRS-Bouillon, ggf. Magermilchpulver und Chlorophyllin-Kupfer-Komplex lag bei *Lactobacillus sp.* DSM 26912 und *Lb. rhamnosus* ATCC 7469 bei $1,1x10^7$ KbE/ml bzw. $3,0x10^7$ KbE/ml.

Nach einer Lagerung von 6 h bei Raumtemperatur lag der Keimgehalt in der Rezeptur 1001 bei $3,2x10^6$ KbE/ml für DSM 26912 und bei $7,0x10^6$ KbE/ml für *Lb. rhamnosus* ATCC 7469. Mit einem Zusatz von 2 % Magermilchpulver (Rezeptur 1002) lag der Keimgehalt nach 6 h bei $9,6x10^6$ KbE/ml für DSM 26912 und bei $1,6x10^7$ KbE/ml für *Lb. rhamnosus* ATCC 7469. Mit abnehmender Zugabe des Magermilchpulvers verringerte sich die Überlebensfähigkeit der Keime, so dass für die Feldstudie als Zitzentauchmittel eine inkubierte, durch Zentrifugation aufkonzentrierte MRS-Bouillon mit einem Zusatz von 2 % Magermilchpulver und 0,1 % Chlorophyllin-Kupfer-Komplex (Rezeptur 1002) ausgewählt wurde. Bei dem Stämmen DSM 26911 und *Lc. lactis* ATCC 11454 lag die Ausgangskeimzahl vor der Lagerung bei $< 1,0x10^6$ KbE/ml, eine Verbesserung der Überlebensfähigkeit im Zitzentauchmittel durch einen Zusatz von Magermilchpulver konnte nicht ermittelt werden.

Aufgrund dieser Ergebnisse wurde als Zitzentauchmittel für den Feldversuch ein solches der Rezeptur 1002 verwendet.

Adhäsion von Milchsäurebakterien an das Zitzenkanalepithel

*In vivo*

Die eingesetzten Schutzkulturen waren bis zu vier Tage nach dem Besprühen auf der Zitzenhaut laktierender Kühe nachzuweisen. Bei trockenstehenden Tieren konnten die Milchsäurebakterien auch nach 14 Tagen noch nachgewiesen werden. Bei allen vier Stämmen konnten Keimdichten von bis zu 300 KbE/ml bei vier laktierenden Tieren am Tag 2 und bei einem Tier am Tag 3 nachgewiesen werden. Bei trockenstehenden Tieren konnten für den Stamm DSM 26911 Keimzahlen von bis zu über 250 KbE/ml am Tag 14 realisiert werden. Die Keimzahlen der Stämme DSM 26912 und *Lb. rhamnosus* ATCC 7469 stiegen bei allen untersuchten Vierteln bis zum Tag 14 auf 300 KbE/ml an. Mit dem Stamm *Lc. lactis* ATCC 11454 wurden Keimzahlen von 10 bis 50 KbE/ml erreicht. Damit konnte nachgewiesen werden, dass sich alle in die Untersuchungen einbezogenen Stämme auf der Zitzenhaut sowohl laktierender als auch trockenstehender Tiere etablieren lassen.

*In vitro*

Alle untersuchten Stämme konnten sich an Zellen des Zitzenkanalepithels anlagern (siehe Tabelle 2, Fig.1).

Tabelle 2: Ergebnisse zur Untersuchung der Adhäsion der MSB-Stämme an Zitzenkanalepithelzellen

| Stamm | Adhäsion [%] | Adhäsionsindex |
|---|---|---|
| DSM 26911 | 56,11% | 7,84 |
| DSM 26912 | 81,33% | 13,17 |
| *Lb. rhamnosus ATCC 7469* | 76,19% | 34,13 |
| *Lc. lactis ATCC 11454* | 100,00% | 14,21 |

Die Beobachtungen zeigen, dass sich die Stämme im Zitzenkanal erfolgreich etablieren lassen.

Prophylaktische Wirkung gegen Mastitis (Feldversuch)

Im Rahmen des Feldversuches wurden 170 Färsen vor ihrer Abkalbung mit dem entwickelten Zitzentauchmittel (Rezeptur 1002, wobei das Zitzentauchmittel einen Gesamtkeimgehalt von $2,5 \times 10^8$ bis über $3,0 \times 10^8$ KbE/ml und Keimzahlen für das Isolat 78/37 von $5,6 \times 10^6$ bis über $3,0 \times 10^7$ KbE/ml, für das Isolat 118/37 von $7,9 \times 10^7$ bis über $3,0 \times 10^8$ KbE/ml, für *Lactobacillus rhamnosus* ATCC 7469 von $1,8 \times 10^8$ bis über $3,0 \times 10^8$ KbE/ml und für *Lactococcus lactis* ATCC 11454 von $1,8 \times 10^8$ bis über $3,0 \times 10^8$ KbE/ml erreichte) auf der Basis von lebenden Milchsäurebakterien im Split-Udder-Design gedippt.

Während zwischen keinem und einmaligem Dippen im Wochenabstand noch kein signifikanter Unterschied in Bezug auf die Anzahl mit S. *aureus* oder mit sogenannten "major pathogens" (*S. aureus, Sc. dysgalactiae, Sc. uberis*) infizierten Eutervierteln zu ermitteln war, zeigte der Vergleich der mehr als zweimal gedippten Viertel mit den nur einmal oder nicht gedippten Vierteln einen signifikanten Unterschied in der Infektionsrate (0 oder 1 Dipp = 4,5 % Major pathogens (Mp) und 3,8 % *S. aureus*; >1 Dipp = 0,9 % Mp und 0,9 % S. aureus (P < 0,0001)). Auch in Bezug auf die somatische Zellzahl war festzustellen, dass bei mehr als einer durchgeführten Anwendung signifikant mehr Euterviertel in beiden Kontrolluntersuchungen weniger als 100.000 Zellen/ml aufwiesen (0 oder 1 Dipp = 38,7 % Viertel < 100.000 Zellen in Kontrolle 1+2; >1 Dipp 44,4 % (P < 0,0137)). Weiterhin erkrankten in der nicht oder einmal gedippten Gruppe zwischen der ersten und der zweiten Kontrolluntersuchung 9,7 % der Viertel neu (Zellzahlüberschreitung > 100.000 Zellen/ml) und in der mehrfach gedippten Gruppe 6,5 % der Viertel (P 0,0128).

Die durchgeführten Untersuchungen zeigen, dass durch die mehrmalige Anwendung des entwickelten Zitzentauchmittels ein erheblicher Einfluss auf die Eutergesundheit der Tiere genommen werden kann. Während mit einer einmaligen Anwendung noch keine sichere Verdrängung einer pathogenen Flora oder/und ein sicherer Schutz vor Neuinfektionen erreicht werden kann, sorgt die mehrmalige Anwendung für eine protektive Flora im Zitzenkanal und damit für eine Verringerung der Neuinfektionsrate.

Die erfolgreiche Entwicklung des innovativen Zitzentauchmittels auf mikrobiologischer Basis stellt eine bedeutende Maßnahme zur Reduzierung des Desinfektionsmitteleinsatzes in der Milcherzeugung dar. Durch den Beitrag zur Verbesserung der Eutergesundheit kann die Anwendung antibiotischer Therapien reduziert werden. Dies wirkt der Entstehung von Antibiotika-Resistenzen entgegen, vermindert das Risiko von Rückstandsproblematiken und erhöht die Lebensmittelsicherheit.

Es konnte gezeigt werden, dass sich mikrobielle Populationen der Zitze gezielt beeinflussen lassen. Milchsäurebakterien, die hemmend auf Mastitiserreger wirken, ließen sich erfolgreich auf den Zitzenepithelien etablieren. Durch Verwendung geeigneter Milchsäurebakterien kann, wie die Anwendungsversuche gezeigt haben, die Färsenmastitisrate deutlich reduziert werden. Störungen der Zitzenhautkondition oder andere Einschränkungen der Verträglichkeit waren nicht festzustellen. Zusammenfassend gelang die Etablierung der erfindungsgemäßen probiotischen Stämme auf den Zitzenepithelien. In Kombination mit der hemmenden Wirkung auf euterpathogene Mikroorganismen konnte eine Verdrängung von Mastitiserregern erzielt und damit nachhaltig die Eutergesundheit verbessert werden.

Die Anwendung eines innovativen Zitzentauchmittels, dessen Mastitiserreger abtötende Wirkung ausschließlich auf mikrobiologischen Komponenten beruht, bringt zwei wesentliche Vorteile mit sich:

1. Die Eutergesundheit in Milchviehbetrieben wird positiv beeinflusst.
2. Aufgrund einer verringerten Mastitisrate wird der Antibiotika-Einsatz in der Milchviehhaltung nachhaltig reduziert.

Damit tragen diese Ergebnisse nicht nur aktiv zur Verbesserung der Tiergesundheit, sondern auch zur Erhöhung der Lebensmittelsicherheit und damit zum Verbraucherschutz bei. Das Risiko für die Entstehung von Antibiotika-Resistenzen in Folge konventioneller Antibiotika-Therapien kann maßgeblich reduziert werden.

Aus den Ergebnissen ergeben sich folgende, praxisrelevante Schlussfolgerungen:

1. Milchsäurebakterien, die Mastitiserreger abtöten oder hemmen, können auf den Zitzenepithelien erfolgreich etabliert werden.
2. Milchsäurebakterien können nach Anpassung der Rezeptur mehrere Tage in einem Zitzentauchmittel überleben.
3. Die Anwendung eines Zitzentauchmittels mit Schutzkulturen reduziert die Färsenmastitisrate und bewirkt somit eine verbesserte Eutergesundheit nach der Abkalbung.

## Patentansprüche

1. Probiotischer Stamm, **dadurch gekennzeichnet, dass** der Stamm ausgewählt ist aus der Gruppe *Lactobacillus* sp. DSM 26911 hinterlegt bei der DSMZ unter der Eingangsnummer 26911, *Lactobacillus* sp. DSM 26912 hinterlegt bei der DSMZ unter der Eingangsnummer 26912 und *Lactobacillus* sp. DSM 26913 hinterlegt bei der DSMZ unter der Eingangsnummer 26913.

2. Zusammensetzung enthaltend mindestens zwei probiotische Stämme, **dadurch gekennzeichnet, dass** mindestens ein Stamm davon einer ist aus *Lactobacillus* sp. DSM 26911 hinterlegt bei der DSMZ unter der Eingangsnummer 26911, *Lactobacillus* sp. DSM 26912 hinterlegt bei der DSMZ unter der Eingangsnummer 26912 oder *Lactobacillus* sp. DSM 26913 hinterlegt bei der DSMZ unter der Eingangsnummer 26913 ist.

3. Zusammensetzung nach Anspruch 2 **dadurch gekennzeichnet, dass** der oder die weiteren Stämme mindestens einer ist aus *Lactococcus lactis* ATCC 11454 und *Lactobacillus rhamnosus* ATCC 7469.

**4.** Zusammensetzung nach einem der Ansprüche 2 oder 3, **dadurch gekennzeichnet, dass** diese; i) die probiotischen Stämme *Lactococcus lactis* ATCC 11454 und *Lactobacillus* sp. DSM 26911; ii) die probiotischen Stämme *Lactococcus lactis* ATCC 11454 und *Lactobacillus* sp. DSM 26912; iii) die probiotischen Stämme *Lactobacillus rhamnosus* ATCC 7469 und *Lactobacillus* sp. DSM 26911; iv) die probiotischen Stämme *Lactobacillus* sp. DSM 26911 und *Lactobacillus* sp. DSM 26912; v) die probiotischen Stämme *Lactobacillus* sp. DSM 26911 und *Lactobacillus* sp. DSM 26912; oder vi) die probiotischen Stämme *Lactobacillus* sp. DSM 26911, *Lactobacillus* sp. DSM 26912, *Lactobacillus rhamnosus* ATCC 7469 und *Lactococcus lactis* ATCC 11454 enthält.

**5.** Pharmazeutisches Mittel enthaltend mindestens einen probiotischen Stamm nach Anspruch 1 oder mindestens eine Zusammensetzung nach einem der Ansprüche 2 bis 4.

**6.** Pharmazeutisches Mittel nach Anspruch 5 formuliert für die Verwendung in der Prophylaxe und/oder Therapie von Mastitis.

**7.** Pharmazeutisches Mittel nach einem der Ansprüche 5 oder 6 formuliert als Zitzentauchmittel oder Sprühmittel.

**8.** Pharmazeutisches Mittel nach einem der Ansprüche 5 bis 7, wobei die enthaltenen Mikroorganismen in gefrorener, lyophylisierter oder getrockneter Form vorliegen.

**9.** Probiotischer Stamm nach Anspruch 1 oder eine Zusammensetzung nach einen der Ansprüche 2 bis 4 zur Verwendung in der Prophylaxe und/oder Therapie von mikrobiellen Infektionen oder Kolonisierung von Mikroorganismen.

**10.** Probiotischer Stamm nach Anspruch 1 oder eine Zusammensetzung nach einen der Ansprüche 2 bis 4 zur Verwendung der Prophylaxe und/oder Therapie von Mastitis.

**11.** Verwendung von mindestens einem probiotischen Stamm nach Anspruch 1 oder mindestens einer Zusammensetzung nach einem der Ansprüche 2 bis 4 als kosmetisches Mittel.

**12.** Verwendung nach Anspruch 11 als kosmetisches Mittel für Tiere, insbesondere Kühe.

**13.** Pharmazeutisches Mittel nach einem der Ansprüche 5 bis 8 zur Verwendung in der Prophylaxe und/oder Therapie von mikrobiellen Infektionen oder Kolonisierung von Mikroorganismen.

## Claims

**1.** Probiotic strain, **characterized in that** the strain is selected from the group consisting of *Lactobacillus* sp. DSM 26911 deposited at the DSMZ under the accession number 26911, *Lactobacillus* sp. DSM 26912 deposited at the DSMZ under the accession number 26912 and *Lactobacillus* sp. DSM 26913 deposited at the DSMZ under the accession number 26913.

**2.** Composition containing at least two probiotic strains, **characterized in that** at least one strain thereof is is one from *Lactobacillus* sp. DSM 26911 deposited at the DSMZ under the accession number 26911, *Lactobacillus* sp. DSM 26912 deposited at the DSMZ under the accession number 26912 or *Lactobacillus* sp. DSM 26913 deposited at the DSMZ under the accession number 26913.

**3.** Composition according to Claim 2, **characterized in that** the further strain(s) is/are at least one from *Lactococcus lactis* ATCC 11454 and *Lactobacillus rhamnosus* ATCC 7469.

**4.** Composition according to either of Claims 2 and 3, **characterized in that** it contains: i) the probiotic strains *Lactococcus lactis* ATCC 11454 and *Lactobacillus* sp. DSM 26911; ii) the probiotic strains *Lactococcus lactis* ATCC 11454 and *Lactobacillus* sp. DSM 26912; iii) the probiotic strains *Lactobacillus rhamnosus* ATCC 7469 and *Lactobacillus* sp. DSM 26911; iv) the probiotic strains *Lactobacillus* sp. DSM 26911 and *Lactobacillus* sp. DSM 26912; v) the probiotic strains *Lactobacillus* sp. DSM 26911 and *Lactobacillus* sp. DSM 26912; or vi) the probiotic strains *Lactobacillus* sp. DSM 26911, *Lactobacillus* sp. DSM 26912, *Lactobacillus rhamnosus* ATCC 7469 and *Lactococcus lactis* ATCC 11454.

5. Pharmaceutical product containing at least one probiotic strain according to Claim 1 or at least one composition according to any of Claims 2 to 4.

6. Pharmaceutical product according to Claim 5 formulated for use in the prophylaxis and/or therapy of mastitis.

7. Pharmaceutical product according to either of Claims 5 and 6 formulated as a teat dip or a spray.

8. Pharmaceutical product according to any of Claims 5 to 7, wherein the microorganisms present are in frozen, lyophilized or dried form.

9. Probiotic strain according to Claim 1 or a composition according to any of Claims 2 to 4 for use in the prophylaxis and/or therapy of microbial infections or colonization of microorganisms.

10. Probiotic strain according to Claim 1 or a composition according to any of Claims 2 to 4 for use the prophylaxis and/or therapy of mastitis.

11. Use of at least one probiotic strain according to Claim 1 or of at least one composition according to any of Claims 2 to 4 as cosmetic product.

12. Use according to Claim 11 as cosmetic product for animals, more particularly cows.

13. Pharmaceutical product according to any of Claims 5 to 8 for use in the prophylaxis and/or therapy of microbial infections or colonization of microorganisms.


**Revendications**

1. Souche probiotique, **caractérisée en ce que** la souche est choisie dans le groupe constitué par *Lactobacillus* sp. DSM 26911 déposé auprès du DSMZ sous le numéro de dépôt 26911, *Lactobacillus* sp. DSM 26912 déposé auprès du DSMZ sous le numéro de dépôt 26912 et *Lactobacillus* sp. DSM 26913 déposé auprès du DSMZ sous le numéro de dépôt 26913.

2. Composition contenant au moins deux souches probiotiques, **caractérisée en ce qu'**au moins une souche est est *Lactobacillus* sp. DSM 26911 déposé auprès du DSMZ sous le numéro de dépôt 26911, *Lactobacillus* sp. DSM 26912 déposé auprès du DSMZ sous le numéro de dépôt 26912 ou *Lactobacillus* sp. DSM 26913 déposé auprès du DSMZ sous le numéro de dépôt 26913.

3. Composition selon la revendication 2, **caractérisée en ce que** la ou les autres souches sont au moins une choisie parmi *Lactococcus lactis* ATCC 11454 et *Lactobacillus rhamnosus* ATCC 7469.

4. Composition selon l'une quelconque des revendications 2 ou 3, **caractérisée en ce que** celle-ci contient : i) les souches probiotiques *Lactococcus lactis* ATCC 11454 et *Lactobacillus* sp. DSM 26911 ; ii) les souches probiotiques *Lactococcus lactis* ATCC 11454 et *Lactobacillus* sp. DSM 26912 ; iii) les souches probiotiques *Lactobacillus rhamnosus* ATCC 7469 et *Lactobacillus* sp. DSM 26911 ; iv) les souches probiotiques *Lactobacillus* sp. DSM 26911 et *Lactobacillus* sp. DSM 26912 ; v) les souches probiotiques *Lactobacillus* sp. DSM 26911 et *Lactobacillus* sp. DSM 26912 ; ou vi) les souches probiotiques *Lactobacillus* sp. DSM 26911, *Lactobacillus* sp. DSM 26912, *Lactobacillus rhamnosus* ATCC 7469 et *Lactococcus lactis* ATCC 11454.

5. Agent pharmaceutique contenant au moins une souche probiotique selon la revendication 1 ou au moins une composition selon l'une quelconque des revendications 2 à 4.

6. Agent pharmaceutique selon la revendication 5, formulé pour l'utilisation dans la prophylaxie et/ou le traitement de la mastite.

7. Agent pharmaceutique selon l'une quelconque des revendications 5 ou 6, formulé en tant qu'agent de trempage des trayons ou agent de pulvérisation.

8. Agent pharmaceutique selon l'une quelconque des revendications 5 à 7, dans lequel les microorganismes contenus sont présents sous forme congelée, lyophilisée ou séchée.

9. Souche probiotique selon la revendication 1 ou composition selon l'une quelconque des revendications 2 à 4, destinée à une utilisation dans la prophylaxie et/ou le traitement d'infections microbiennes ou d'une colonisation par des microorganismes.

10. Souche probiotique selon la revendication 1 ou composition selon l'une quelconque des revendications 2 à 4, destinée à une utilisation la prophylaxie et/ou le traitement de la mastite.

11. Utilisation d'au moins une souche probiotique selon la revendication 1 ou d'au moins une composition selon l'une quelconque des revendications 2 à 4 en tant qu'agent cosmétique.

12. Utilisation selon la revendication 11 en tant qu'agent cosmétique pour des animaux, notamment des vaches.

13. Agent pharmaceutique selon l'une quelconque des revendications 5 à 8, destiné à une utilisation dans la prophylaxie et/ou le traitement d'infections microbiennes ou d'une colonisation par des microorganismes.

Fig.1 von 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005034970 A1

- WO 2008145756 A1


**In der Beschreibung aufgeführte Nicht-Patentliteratur**

- Deutsche Veterinärmedizinische Gesellschaft (DVG), 2002: Leitlinien zur Bekämpfung der Mastitis des Rindes als Bestandsproblem. Kurzes Lehrbuch der Milchkunde und der Milchhygiene. MVS Medizinverlage, 2007
- **SMITH et al.** *J.Dairy. Sci.,* 1985, vol. 68, 402-417
- **MALLARD et al.** *J. Dairy Sci.,* 1998, vol. 81, 585-595
- **GREEN et al.** *J. Dairy Sci.,* 2002, vol. 85, 2589-2599
- Kurzes Lehrbuch der Milchkunde und der Milchhygiene. MVS Medizinverlage, 2007
- **HERTL et al.** *J. Dairy Sci.,* 2011, vol. 94, 4863-4877
- **DIEZ-GONZALEZ.** *Curr. Issues Intestinal Microbiol.,* 2007, vol. 8, 15-24
- **ESPECHE et al.** *Anaerobe,* 2012, vol. 18 (1), 103-109
- **DE MAN et al.** *J. Appl. Bact.,* 1960, vol. 23, 130-135
- **MAN et al.** *J. Appl. Bact.,* 1960, vol. 23, 130-135

- Genus Leuconostoc. **GARVIE EI.** Bergey's Manual of Systematic Bacteriology. 1986, vol. 2, 1071-1075
- Genus Pediococcus. **GARVIE EI.** Bergey's Manual of Systematic Bacteriology. 1986, vol. 2, 1075-1079
- Genus Streptococcus. **HARDIE, JM.** Bergey's Manual of Systematic Bacteriology. 1986, vol. 2, 1043-1071
- Genus Lactobacillus. **KANDLER O ; WEISS N.** Bergey's Manual of Systematic Bacteriology. 1986, vol. 2, 1209-1234
- **ANLEHNUNG ; RYAN et al.** *Appl. Environ. Microbiol.,* 1996, vol. 62, 612-619
- *Appl. Environ. Microbiol.,* 1998, vol. 64, 2287-2290
- **MATHOT et al.** *J. Dairy Sci.,* 2003, vol. 86, 3068-3074
- **ANLEHNUNG ; FROLA et al.** *J. Dairy Res.,* 2011, vol. 14, 1-9
- **PADUCH ; KRÖMKER.** Tierärztl Prax. 2011, vol. 39, 71-76